# FASCICULE DE BREVET EUROPEEN

(11) **EP 0 650 374 B1**
(45) Date de publication et mention de la délivrance du brevet: **26.07.2000**
(21) Numéro de dépôt: 94916275.4
(22) Date de dépôt: 11.05.1994
(51) Int. Cl.: A61L 27/00, A61K 48/00

(54) **UTILISATION D'UN MATERIAU POREUX A BASE DE CARBONATE DE CALCIUM COMME SUPPORT POUR UN FACTEUR DE CROISSANCE DANS LA PREPARATION D'UN IMPLANT BIORESORBABLE**
VERWENDUNG EINES PORÖSEN MATERIALS AUS KALZIUMCARBONAT ALS TRÄGER FÜR EINEN WACHSTUMSFAKTOR IN DER HERSTELLUNG EINES RESORBIERBAREN IMPLANTATES
USE OF A CALCIUM CARBONATE BASED POROUS MATERIAL AS SUPPORT FOR A GROWTH FACTOR IN THE PREPARATION OF A BIOABSORBABLE IMPLANT

(30) Priorité: 13.05.1993 FR 9305783; 17.11.1993 FR 9313740
(43) Date de publication de la demande: 03.05.1995
(73) Titulaire: BIO HOLDINGS INTERNATIONAL LIMITED, Tortola (VG)
(72) Inventeur: PATAT, Jean-Louis, F-75017 Paris (FR); OUHAYOUN, Jean-Pierre, F-75006 Paris (FR)
(74) Mandataire: Hubert, Philippe
(86) Numéro de dépôt international: FR9400565
(87) Numéro de publication internationale: WO9426322

(56) Documents cités:
- EP-A- 0 395 187
- WO-A-86/01726
- DE-A- 3 542 744
- DE-A- 4 130 546
- FR-A- 2 637 502
- US-A- 3 919 971
- MEDICINE SCIENCES, vol.9, no.2, Février 1993 pages 208 - 210 O. DANOS 'Réimplantation de cellules génétiquement modifiées dans des néo-organes vascularisés; ed. John Libbey Eurotext; FR'
- JOURNAL OF CELLULAR BIOCHEMISTRY supplément 15F, 1-7 avril 1991, abrégé CF15, ed. Wiley-Liss, P. MOULLIER ET AL. 'Organoid neovascular structure: effects of various matrix and angiogenic factors'

## Description

L'invention a pour objet l'utilisation d'un matériau à base de carbonate de calcium comme support pour un facteur de croissance dans la préparation d'un implant biorésorbable destiné à être mis en place dans un organisme vivant.

Un tel matériau peut être utilisé notamment comme implant ostéoformateur.

On sait que la chirurgie osseuse nécessite souvent la mise en place de greffons osseux ou d'implants constituant des produits de remplacement artificiels de tels greffons.

La réalisation d'allogreffes soulève des objections, pour des raisons évidentes de santé publique, compte-tenu des risques de transmission de certaines maladies virales paves ou de maladies provoquées par des agents transmissibles non conventionnels ou "prions".

De ce point de vue, la réalisation d'autogreffes est plus satisfaisante, mais le prélèvement du greffon entraîne des risques de morbidité importants ; voir par exemple Christopher J. Damien et J. Russell Parsons, Journal of Applied Biomaterials, Vol.2, 187-208 (1991).

Pour ces raisons, on a préconisé l'utilisation d'implants à base de matériaux biocompatibles, comme le phosphate tricalcique, l'hydroxyapatite, le plâtre, le corail, les polymères à base de poly(acide lactique) et/ou poly(acide glycolique), etc.

Certains de ces matériaux, dont le carbonate de calcium, sont biorésorbables et permettent la formation progressive de tissu osseux néoformé aux dépens du matériau implanté en voie de résorption ; voir notamment le brevet européen 0 022 724.

On sait par ailleurs que la présence de certains facteurs ostéoinducteurs dans les implants favorise la repousse osseuse. Toutefois, les spécialistes estimaient jusqu'à présent qu'il était nécessaire d'ajouter à l'implant du collagène servant de support pour les facteurs ostéoinducteurs ; voir à ce sujet l'article de Damien et Russell Parsons déjà cité ci-dessus et la demande de brevet FR-2.637.502.

On a maintenant découvert qu'un matériau à base de carbonate de calcium poreux, tel que le corail, peut servir de support à des facteurs ostéoinducteurs, et plus généralement à des facteurs de croissance, dans la préparation d'implants biorésorbables et que la présence de collagène n'est ni nécessaire ni souhaitable dans le cas où l'implant est destiné à être utilisé comme implant ostéoformateur..

La présente invention a donc pour objet l'utilisation d'un matériau poreux constitué essentiellement de carbonate de calcium, y compris en surface, comme support pour au moins un facteur de croissance, ledit support étant exempt de collagène dans le cas où ledit facteur de croissance est un facteur ostéoinducteur, dans la préparation d'un implant biorésorbable destiné à être mis en place dans un organisme animal vivant, en particulier chez un animal vertébré, y compris les humains.

Lorsque les facteurs de croissance sont des facteurs ostéoinducteurs, les implants sont utilisables comme implants ostéoformateurs. Bien entendu, ces implants peuvent contenir, outre les facteurs ostéoinducteurs, d'autres facteurs de croissance. Les implants ainsi obtenus peuvent être sommis en place comme pièces de comblement osseux progressivement résorbables au profit d'un tissu néoformé, soit implantés dans un site non osseux, par exemple un tissu conjonctif, où ils donneront naissance à un tissu osseux utilisable ultérieurement comme matériau d'autogreffe osseuse.

Lorsque le support de carbonate de calcium est chargé avec un facteur de croissance non ostéoinducteur, il peut être utilisé notamment comme support pour la culture *in vivo* de cellules vivantes. Selon les cellules et les facteurs de croissance utilisés, l'implant, après mise en place, peut servir notamment de support pour l'obtention d'un tissu néoformé sur le lieu d'implantation. Ce tissu néoformé peut être utilisé comme élément de remplacement de parties d'organe défaillant (raccord pancréas-intestin, urêtre, vessie, péricarde, etc).

L'implant de l'invention peut également être utilisé comme support de culture *in vivo* pour des cellules modifiées génétiquement, notamment par insertion d'un gène approprié, de façon connue en soi, pour remédier à un défaut génétique. Les cellules modifiées peuvent éventuellement provenir d'un prélèvement autologue. Les implants ainsi obtenus constituent un "organoïde" servant de dispositif de traitement thérapeutique dont la mise en place permet de suppléer un organe défaillant, et en particulier de remédier à certains dysfonctionnements d'origine génétique. Il s'agit donc d'un nouveau mode de mise en oeuvre des thérapies géniques.

Par exemple, on peut réaliser une pièce creuse en corail, munie d'une ouverture obturable par un bouchon, vissé ou emboîté, réalisé également en corail. Par exemple, pour l'implantation chez l'homme, il peut s'agir d'un cylindre creux ayant de 2 à 10 cm de haut, de 1 à 3 cm de diamètre externe et de 0,5 à 2 cm de diamètre interne. La paroi interne peut être imprégnée d'un collagène chargé de facteurs de croissance comme le TGF bêta. La paroi externe peut être imprégnée avec une solution d'un facteur angiogénique (FGF). Les cellules autologues (par exemple des fibroblastes) modifiées sont préalablement cultivées *in vitro* sur un support solide comme un réseau de fibres de collagène ou des granules de corail. On introduit dans la partie creuse de l'implant la culture de cellules modifiées, avec son support solide. On obture ensuite à l'aide du bouchon puis on procède à l'implantation, par exemple intra-abdominale. Les cellules implantées s'organisent en un tissu vascularisé entouré d'un tissu conjonctif remplaçant le corail au fur et à mesure de la résorption de celui-ci.

Les cellules introduites dans l'implant peuvent être des cellules non différenciées dont la différenciation s'effectue grâce à des cytokines endogènes ou exogènes.

Le matériau à base de carbonate de calcium utilisable comme support pour les facteurs de croissance est de préférence de l'aragonite. On peut utiliser en particulier le squelette de corail. L'un des intérêts de l'utilisation du squelette de corail est qu'il contient des pores communiquants, ce qui favorise la néo-vascularisation ainsi que l'envahissement du matériau par les cellules osseuses ou par d'autres cellules introduites préalablement ou recrutées *in situ*.

De préférence, on utilise comme matériau support un matériau ayant des diamètres de pores compris entre 50 et 500 micromètres, en particulier entre 200 et 400 micromètres. La porosité, c'est-à-dire la proportion volumique des pores par rapport au volume total du matériau, est comprise généralement entre 20 et 80 %, par exemple entre 40 et 60 %.

On peut obtenir des matériaux de ce type en utilisant notamment le corail des genres Porites, Acropora, Goniopora, Lobophyllia, Simphyllia et Millipora.

On sait que les facteurs de croissance sont des polypeptides qui agissent localement sur certaines cellules en influençant leur prolifération, leur migration et/ou leur différenciation ou qui stimulent la production d'autres facteurs de croissance.

De nombreux facteurs de croissance sont connus à l'heure actuelle.

Par exemple, les facteurs de croissance ayant un effet ostéoinducteur sont connus et leurs effets ont été étudiés notamment *in vitro* sur des cultures de diverses cellules osseuses.

Parmi les facteurs de croissance dont l'activité est spécifique au milieu osseux, on peut citer certaines protéines de l'os déminéralisé, ou DBM (Demineralized Bone Matrix) et en particulier les protéines appelées BP (Bone Protein) ou BMP (Bone Morphogenetic Protein), qui contiennent en fait plusieurs constituants ; chez l'homme, par exemple, on a isolé notamment les constituants appelés ostéonectine et ostéocalcine. Une autre protéine active est l'ostéogénine.

Parmi les facteurs de croissance influençant la croissance cellulaires en général, y compris la croissance des cellules osseuses, on peut citer notamment les somatomédines ou IGF (Insulin-like Growth Factor), le PDGF (Platelet-Derived Growth Factor), le FGF (Fibroblast Growth Factor), le BDGF II (ou bêta-2-microglobuline), les TGF (Transforming Growth Factor), en particulier le TGF bêta (ou bTGF), etc.

Les doses de facteurs de croissances utilisables sont connues ou peuvent être déterminées par des expériences de routine sur des modèles animaux. Généralement, on utilise les facteurs de croissance, dont l'action est locale, à des doses de quelques microgrammes ou de quelques dizaines de microgrammes.

L'invention concerne également la préparation des supports de carbonate de calcium poreux chargés de facteurs de croissance. Ces derniers peuvent être incorporés :
- par imprégnation, suivie ou non de séchage, à l'aide d'une solution ou d'un gel contenant le facteur de croissance,
- par dépôt de films par capillarité à l'aide d'une solution du facteur de croissance par liaison avec le carbonate de calcium par l'intermédiaire d'un revêtement à potentiel de surface électronégatif (héparine, sulfate d'héparane, sulfate de dextrane, sulfate de dermatane, sulfate de xylane, etc).

Il est possible de faciliter la fixation et la rétention des facteurs de croissance sur le support de carbonate de calcium en les appliquant sous la forme d'une association avec un agent liant, notamment un agent liant capable de former un gel. De tels agents liants sont connus. Il s'agit par exemple du collagène ou de dérivés de cellulose comme la méthylcellulose, l'hydroxypropylcellulose, l'hydroxypropylméthylcellulose, la carboxyméthylcellulose, etc. Ces produits sont biocompatibles. Ils sont disponibles commercialement. On peut encore utiliser comme agents liants un précurseur de la fibrine, par exemple du fibrinogène ou une colle biologique (cryoprécipité).

L'agent liant a notamment pour effet de favoriser la fixation du facteur de croissance et éventuellement son relargage progressif.

Généralement, l'agent liant est progressivement résorbé au fur et à mesure de la réhabitation de l'implant par un tissu néoformé.

On a cependant constaté que, dans le cas d'un implant ostéoformateur, la présence de collagène comme agent liant ne favorise pas la création d'un nouveau tissu osseux, mais aurait au contraire tendance à la retarder.

Les exemples suivants illustrent l'invention.

### EXEMPLE 1 :

On utilise comme matériau de départ des disques de corail ayant un diamètre de 6 mm et une épaisseur de 2 mm (origine INOTEB).

On utilise comme facteur ostéoinducteur une BP bovine ayant une concentration en protéines de 1,52 mg/ml.

On dilue la BP de façon à obtenir des doses de 10, 35 et 100 microgrammes de principe actif sous un volume de 20 microlitres. On imprègne chaque disque de corail avec 20 microlitres de l'une des solutions obtenues. Après infiltration de la solution dans les pores, les disques sont congelés rapidement puis soumis à une lyophilisation.

Avec les disques ainsi traités, on réalise des implants sous-cutanés dans la région ventrale chez des rats. On utilise trois groupes de cinq animaux qui reçoivent chacun un implant de corail imprégné de 10, 35 ou 100 microgrammes de BP, respectivement.

Au bout de 27 jours, on administre aux animaux, par voie sous-cutanée, une dose de 20 mg/kg d'un agent marqueur constitué de calcéine. Le vingt-huitième jour, les implants sont extraits, pesés, photographiés et fixés dans le méthanol. Les échantillons sont enrobés dans du poly(méthacrylate de méthyle) en vue de l'examen histologique. Les échantillons sont coupés et colorés avec le réactif Rapid Bone Stain et avec de la picro-fuchsine Van Gieson. Les coupes sont examinées au microscope.

Les poids des disques de corail avant implantation et après explantation sont indiqués dans le tableau 1 suivant, dans lequel les résultats sont exprimés sous la forme d'une moyenne ± écart-type.

**TABLEAU 1**

| Groupe | Poids des disques (mg) | | Gain de poids |
|---|---|---|---|
| | avant implantation | après explantation | |
| I | 86,0±11,2 | 97,2±13,0 | 11,2±6,0 |
| II | 84,4±4,1 | 114,2±14,2 | 29,8±12,1 |
| III | 83,2±9,5 | 128±12,2 | 45,4±10,1 |

Dans le groupe I, l'examen au microscope montre une formation d'os encore immature et de moelle hématopoïétique à travers tout l'implant, ainsi que la formation d'os en surface. L'os se forme et la conduction par le matériau permet la croissance de l'os. Il y a une harmonie entre l'ostéoinduction et l'ostéoconduction.

Dans le groupe II, la minéralisation est bonne dans l'ensemble de l'implant, l'os néoformé est mature et tapisse le corail. L'ostéoinduction domine l'ostéoconduction et l'os est plus important sur le pourtour de l'implant.

Dans le groupe III, les échantillons ont un bord externe épais d'os mature avec de la moelle hématopoïétique au centre. Dans plusieurs cas, du cartilage et du cartilage en voie de minéralisation sont observés au bout de quelques semaines.

### EXEMPLE 2 :

On procède sur des rats à une trépanation par ablation d'un volet circulaire de 8 mm de diamètre sur le dessus du crâne. On applique dans la fenêtre ainsi obtenue un disque de corail ayant de 8 mm de diamètre. Les disques de corail sont imprégnés, de façon analogue à celle décrite à l'exemple précédent, par le facteur ostéoinducteur (BMP-2 humaine recombinante), à raison de 0,5 microgramme de BMP-2 pour 1 mg de corail. Le jour de l'opération, on injecte par voie intramusculaire de la calcéine à raison de 20 mg/kg. Au vingt-septième jour, on injecte par voie intramusculaire du xylènol orange (90 mg/kg).

Ces colorants donnent une fluorescence jaune et orange délimitant les formations osseuses.

Au vingt-huitième jour, on prélève une partie de la calotte crânienne, incluant l'implant, et on la fixe dans le formol neutre à 10 % pour étude histologique après inclusion dans le méthacrylate de méthyle sur coupes non déminéralisées colorées avec le colorant Giemsa-Paragon. Des microradiographies mettent en évidence la minéralisation.

Les coupes montrent une formation osseuse à partir des berges de la lésion osseuse et s'étendant sur les bords du disque de corail

Dans le cas du corail imprégné de BMP-2, de l'os est présent sur l'ensemble du disque. La résorption du corail est plus rapide qu'en absence de BMP-2.

### EXEMPLE 3 :

On procède sur des lapins à une trépanation bilatérale et symétrique du crâne au niveau des os pariétaux, par ablation de deux volets circulaires de 10 mm de diamètre.

On constitue cinq lots de lapins : quatre groupes de test et un poupe témoin. Sur les individus de chaque groupe de test, un seul côté est traité.
Groupe 1 (Symbole MF) : on applique dans l'une des fenêtres 50 microlitres de gel de méthylcellulose contenant 1 µg de TGF bêta.
Groupe 2 (Symbole BF) : on applique dans l'une des fenêtres 100 µl de colle biologique à laquelle on a ajouté 1 µg de TGF bêta.
Groupe 3 (Symbole BCF) : on applique 50 microlitres d'un mélange analogue à celui appliqué au groupe BF, mais contenant en outre 70 mg de granulés de corail *Porites*, commercialisés sous la dénomination BIOCORAL 450 par la Société INOTEB.
Groupe 4 (Symbole BC) : on applique le même mélange que pour le poupe 3, mais sans facteur de croissance.

On suit l'évolution de la construction osseuse par tomodensitométrie crânienne. Les diamètres et surface des trépanations sont mesurées sur un analyseur d'images. Après le sacrifice des animaux étudiés, au bout d'un mois, la calotte crânienne est prélevée puis préparée avec un microtome pour l'analyse histologique par histomorphométrie et microscopie en fluorescence. Pour marquer les surfaces d'ostéoformation, on a injecté 2 ml d'oxytétraquinol aux dixième et troisième jours précédant le sacrifice.

On a fait les observations suivantes :

Au bout de 30 jours, les groupes BC et BCF ont complètement recouvert la cicatrice par un tissu ostéoïde.

Pour les autres groupes, le recouvrement n'est pas complet. Le diamètre du côté traité est inférieur au diamètre du côté témoin. Le diamètre du côté témoin est inférieur aux diamètres observés dans le groupe témoin.

Le volume des travées osseuses (VTO) a été déterminé par histomorphométrie.

Le poupe BCF a le VTO le plus élevé, suivi par le poupe BC.

L'étude des animaux des groupes traités avec du corail montre que les granulés sont résorbés progressivement. Le corail a permis la création rapide de travées osseuses entre ces granulés.

D'une façon générale, l'adjonction du facteur de croissance élève la vitesse de reminéralisation.

### EXEMPLE 4 :

On recouvre une pièce de corail (porosité : 50 %) avec un gel de collagène murin de type I contenant un facteur de croissance angiogénique (bFGF).

En utilisant un bFGF marqué à l'iode radioactif, on a pu montrer, par des essais de relargage, que la liaison du bFGF avec le support de corail est très forte. Cette liaison est encore augmentée en présence d'héparanes ou d'héparines.

Les pièces de corail ainsi traitées sont implantées en site sous-cutané chez des souris. On procède durant un an à l'observation macroscopique et histologique des implants.

En quelques mois, le corail est résorbé. Il laisse place à un tissu conjonctif de même taille et de de même forme que l'implant. Au bout de trois mois, on observe une forte vascularisation et l'absence de toute cellule inflammatoire en regard des résidus de corail. Le tissu conjonctif est formé de cellules mésenchymateuses peu denses.

Dans d'autres expériences, on a remplacé le collagène par de la carboxyméthylcellulose ou du fibrinogène.

### EXEMPLE 5 :

On réalise un cylindre creux en corail.

Dans la partie creuse, on introduit un réseau de fibres de collagène contenant des fibroblastes autologues génétiquement modifiés par insertion d'un gène à l'aide d'un vecteur rétroviral.

Préalablement, on a imprégné la paroi externe du cylindre avec un gel de collagène de type I associé à de l'héparine. Le corail ainsi prétraité est incubé en présence d'un facteur de croissance angiogénique (bFGF). Finalement, le cylindre est obturé à l'aide d'un bouchon de corail, puis on effectue une implantation intrapéritonéale, chez un chien de 25 kg.

Au bout de 45 jours, on retire l'implant. La vascularisation de ce dernier est importante et comporte des vaisseaux de gros calibre. La résorption du corail est initiée. Les cellules modifiées sont viables et expriment le gène inséré.

Elles sont organisées en un tissu vascularisé.

On a réalisé une experience analogue sur des souris déficitaires en beta-glucuronidase et présentant une maladie de surcharge lysosomale. Un cDNA humain codant pour la beta-glucuronidase est inséré dans un vecteur rétroviral que l'on utilise ensuite pour modifier des cellules (autologues) de souris. Les cellules modifiées sont introduites dans un réseau de fibres de collagène et l'ensemble est introduit dans un cylindre de corail traité de façon analogue à celle décrite ci-dessus. Les implants ainsi préparés sont insérés dans le mésentère des souris déficientes.

Les cellules modifiées implantées sont capables de sécréter l'enzyme manquante qui passe dans la circulation générale, entraînant ainsi la régression des conséquences physiologiques du défaut génétique, et notamment la correction des lésions hépatiques et spléniques.

## Revendications

1. Utilisation d'un matériau poreux constitué essentiellement de carbonate de calcium, y compris en surface, comme support pour au moins un facteur de croissance, ledit support étant exempt de collagène dans le cas où ledit facteur de croissance est un facteur ostéoinducteur, dans la préparation d'un implant biorésorbable destiné à être mis en place dans un organisme animal vivant, en particulier chez un animal vertébré.

2. Utilisation selon la revendication 1, caractérisée par le fait que ledit carbonate de calcium est sous forme d'aragonite, et en particulier de squelette de corail.

3. Utilisation selon la revendication 1 ou 2, caractérisée par le fait que ledit facteur de croissance est un facteur osréoinducteur.

4. Utilisation selon la revendication précédente, caractérisée par le fait que ledit implant est destiné à être mis en place comme pièce de comblement osseux.

5. Utilisation selon la revendication 3, caractérisée par le fait que ledit implant est destiné à être mis en place dans un site non osseux.

6. Utilisation selon l'une quelconque des revendications 1 et 2, caractérisée par le fait que ledit facteur de croissance est un facteur non ostéoinducteur.

7. Utilisation selon la revendication précédente, caractérisée par le fait que ledit implant est destiné à ta culture de cellules *in vivo*.

8. Utilisation selon la revendication précédente, caractérisée par le fait que lesdites cellules sont des cellules modifiées génétiquement, en particulier des cellules autologues.

9. Utilisation selon la revendication précédente, caractérisée par le fait que lesdites cellules sont modifiées par insertion d'un gène destiné à remédier à un défaut génétique.

10. Utilisation selon l'une quelconque des revendications 7 à 9, caractérisée par le fait que ledit implant se présente sous la forme d'une pièce creuse munie d'une ouverture obturable par un bouchon.

11. Utilisation selon la revendication précédente, caractérisée par le fait qu'au moins un facteur de croissance est déposé par imprégnation sur la paroi interne de ladite pièce creuse.

12. Utilisation selon la revendication 10 ou 11, caractérisée par le fait que l'on introduit dans la partie creuse de l'implant une culture de cellules à cultiver, puis on obture ladite partie creuse à l'aide du bouchon avant de procéder à l'implantation.

## Claims

1. Use of a porous calcium carbonate based material as support for at least one growth factor, the said support being free of collagen in the case where the said growth factor is an osteoinductive factor, in the preparation of a bioabsorbable implant intended to be fitted in a living animal organism, in particular in a vertebrate.

2. Use according to Claim 1, characterised in that the said calcium carbonate is in the form of aragonite, and in particular coral skeleton.

3. Use according to Claim 1 or 2, characterised in that the said growth factor is an osteoinductive factor.

4. Use according to the preceding claim, characterised in that the said implant is intended to be fitted as a bone filler.

5. Use according to Claim 3, characterised in that the said implant is intended to be fitted in a non-osseous site.

6. Use according to either of Claims 1 and 2, characterised in that the said growth factor is a non-osteoinductive factor.

7. Use according to the preceding claim, characterised in that the said implant is intended for in vivo cell culture.

8. Use according to the preceding claim, characterised in that the said cells are genetically modified cells, in particular autologous cells.

9. Use according to the preceding claim, characterised in that the said cells are modified by insertion of a gene intended to remedy a genetic defect.

10. Use according to any one of Claims 7 to 9, characterised in that the said implant is in the form of a hollow piece provided with an opening which can be closed off by a plug.

11. Use according to the preceding claim, characterised in that at least one growth factor is deposited by impregnation on the internal wall of the said hollow piece.

12. Use according to Claim 10 or 11, characterised in that a cell culture to be cultivated is introduced into the hollow part of the implant and the said hollow part is then closed off using the plug before carrying out implantation.

## Patentansprüche

1. Verwendung eines porösen Materials, das im Wesentlichen aus Calciumcarbonat besteht, wobei die Oberfläche eingeschlossen ist, als Träger für mindestens einen Wachstumsfaktor, wobei dieser Träger dann, wenn der Wachstumsfaktor ein osteoinduzierender Faktor ist, collagenfrei ist, für die Herstellung eines biologisch resorbierbaren Implantats, das vorgesehen ist, in einen lebenden tierischen Organismus, insbesondere in ein Wirbeltier, eingepflanzt zu werden.

2. Verwendung nach Anspruch 1**, dadurch gekennzeichnet, dass** das Calciumcarbonat in Form von Aragonit und insbesondere eines Korallenskeletts vorliegt.

3. Verwendung nach Anspruch 1 oder 2**, dadurch gekennzeichnet, dass** der Wachstumstaktor ein osteoinduzierender Faktor ist.

4. Verwendung nach dem vorhergehenden Anspruch**, dadurch gekennzeichnet, dass** das Implantat vorgesehen ist, als Knochenverschlussstück eingepflanzt zu werden.

5. Verwendung nach Anspruch 3**, dadurch gekennzeichnet, dass** das Implantat vorgesehen ist, an einer Stelle eingepflanzt zu werden, die kein Knochen ist.

6. Verwendung nach Anspruch 1 oder 2**, dadurch gekennzeichnet, dass** der Wachstumstaktor ein nicht osteoinduzierender Faktor ist.

7. Verwendung nach dem vorhergehenden Anspruch**, dadurch gekennzeichnet, dass** das Implantat für eine In-vivo-Zellkultur vorgesehen ist.

8. Verwendung nach dem vorhergehenden Anspruch**, dadurch gekennzeichnet, dass** die Zellen genetisch modifizierte Zellen, insbesondere autologe Zellen, sind.

9. Verwendung nach dem vorhergehenden Anspruch**, dadurch gekennzeichnet, dass** die Zellen durch Insertion eines Gens modifiziert sind, das vorgesehen ist, einen genetischen Defekt zu beseitigen.

10. Verwendung nach einem der Ansprüche 7 bis 9**, dadurch gekennzeichnet, dass** das Implantat in Form eines Hohlkörpers vorliegt, der mit einer durch einen Stopfen verschließbaren Öffnung versehen ist.

11. Verwendung nach dem vorhergehenden Anspruch**, dadurch gekennzeichnet, dass** mindestens ein Wachstumsfaktor durch Imprägnieren auf die Innenwand des Hohlkörpers aufgebracht wird.

12. Verwendung nach Anspruch 10 oder 11**, dadurch gekennzeichnet, dass** in den hohlen Teil des Implantats eine Kultur aus zu kultivierenden Zellen eingebracht und dieser hohle Teil anschließend vor der Implantation mittels des Stopfens verschlossen wird.
